# EUROPEAN PATENT SPECIFICATION

(11) **EP 1 976 359 B1**
(45) Date of publication and mention of the grant of the patent: **08.01.2014**
(21) Application number: 08005158.4
(22) Date of filing: 19.03.2008
(51) Int. Cl.: H05K 7/20

(54) **Medical apparatus**
Medizinisches Gerät
Appareil médical

(30) Priority: 30.03.2007 JP 2007094203
(43) Date of publication of application: 01.10.2008
(73) Proprietor: Olympus Medical Systems Corp., Tokyo 151-0072 (JP)
(72) Inventor: Kitahara, Toshihiro, Tokyo 151-0072 (JP)
(74) Representative: von Hellfeld, Axel

(56) References cited:
- JP-A- 2001 340 337
- JP-A- 2006 020 755
- US-A- 5 402 312
- US-A- 5 707 401
- US-B1- 6 450 947
- D.S. GAUNT ET AL: "Cooling Electrical Equipment" IBM TECHNICAL DISCLOSURE BULLETIN, vol. 20, no. 6, 1 November 1977 (1977-11-01), pages 2428-2429, XP001293000

## Description

### BACKGROUND OF THE INVENTION

### 1. Field of the Invention

The present invention relates to a medical apparatus including plural cooling fans, and, more particularly to a medical apparatus used together with an ultrasound endoscope.

### 2. Description of the Related Art

In recent years, an ultrasound diagnostic method for irradiating ultrasound into a body cavity and imaging a state in a body from an echo signal of the ultrasound to perform diagnosis is wide spread. Examples of a medical apparatus used for such an ultrasound diagnostic method include an ultrasound echo apparatus that can image a state in a body from a body surface and an ultrasound endoscope that includes an ultrasound vibrating unit for transmitting and receiving an ultrasound at a distal end portion thereof and can be inserted in a body cavity to image a state in a body.

The ultrasound endoscope is connected to an ultrasound observation apparatus that is a medical apparatus for imaging an echo signal. The ultrasound observation apparatus outputs an electric signal for causing a monitor connected thereto to display an ultrasound image in the body cavity received by the ultrasound endoscope.

In such an ultrasound observation apparatus, as is well known, plural electronic components, a CPU, and the like are built in an armor housing of a box shape. When driven by electric power received from the outside, the electronic components, the CPU, and the like are changed to heat elements and heated by wiring resistance, driving resistance, and the like due to the electric power. Therefore, cooling fans are provided in not only the ultrasound observation apparatus but also a large number of electronic apparatuses.

For such electronic apparatuses including the cooling fans, various proposals have been made in order to improve cooling efficiency. For example, Japanese Patent Application Laid-Open No. 2006-156871 discloses a technique for controlling, in a cooling apparatus in a locker-type housing, interference of cooling fans provided in plural units and efficiently cooling the units.

However, the technique for the cooling apparatus disclosed in Japanese Patent Application Laid-Open No. 2006-156871 is a technique for improving cooling efficiency in a state in which the plural units are superimposed rather than cooling efficiency of a single unit. In one unit, plural heating elements such as electronic components are arrayed in a limited space. Therefore, optimum cooling cannot be performed depending on arrangement positions of the heating elements.

Document JP 2001 340337 discloses ultrasound medical apparatus having a box body separated by a bulk head and cooling fans provided on front and rear surfaces.

US 5,707,401 discloses a medical apparatus having a plurality of cooling fans for cooling an interior of the medical apparatus. A plurality of the cooling fans are arranged in parallel on a first side of the device, and air inlets are arranged on sides of the device adjacent to the first side. Air is drawn by the fans through the air inlets and into the apparatus, and is further exhausted by the fans through air outlets disposed in the vicinity of the fans. A further fan may be disposed in the vicinity of a light source, and may draw air specifically through the light source to be exhausted in the same direction as the air exhausted by the plurality of cooling fans arranged on the first side of the device.

US 5,402,312 discloses a mechanism to cool different parts of an apparatus using different air cooling streams which are separated from each other and which differ from each other with regard to their flow direction. That is, an airflow used to cool chips is used which is different form an airflow used to cool a memory board located below and separated from the chips.

Moreover, in a medical spot, positions of medical apparatuses arranged in an operating room may be restricted in relation to a condition of positions of use in terms of distances to a patient and a surgeon in a limited operating room space. Under such a condition of use of the medical apparatuses, a smaller exhaust volume from the cooling fans is better. The cooling fans have to be arranged to prevent an exhaust direction from the cooling fans from being directed to the patient and the surgeon as much as possible.

The present invention has been devised in view of the circumstances described above and it is an object of the present invention to realize a medical apparatus in which cooling efficiency is improved and that reduces an unpleasant feeling given to a patient and a surgeon because of exhaust from cooling fans.

### SUMMARY OF THE INVENTION

A medical apparatus according to claim 1 is provided. The medical apparatus includes a first heating area in which a first heating unit is provided, a second heating area that is arranged to be superimposed on the first heating area and in which a second heating unit is provided, a first cooling fan that cools the first heating area, and a second cooling fan that has an air current direction substantially orthogonal to an air current direction of the first cooling fan and cools the second heating area.

The above and other objects, feature and advantages of the invention will become more clearly understood from the following description referring to the accompanying drawings.

### BRIEF DESCRIPTION OF THE DRAWINGS

Fig. 1 is a diagram for explaining the schematic configuration of an ultrasound endoscope according to an embodiment of the present invention;
Fig. 2 is a front view of an ultrasound observation apparatus according to the embodiment;
Fig. 3 is a rear view of the ultrasound observation apparatus according to the embodiment;
Fig. 4 is a right side view of the ultrasound observation apparatus according to the embodiment;
Fig. 5 is a left side view of the ultrasound observation apparatus according to the embodiment;
Fig. 6 is a bottom view of the ultrasound observation apparatus according to the embodiment;
Fig. 7 is a perspective view showing the inside of the ultrasound observation apparatus according to the embodiment;
Fig. 8 is a perspective view for explaining air currents in the ultrasound observation apparatus caused by respective cooling fans according to the embodiment;
Fig. 9 is a partial sectional view for explaining air currents in the ultrasound observation apparatus caused by respective cooling fans that cool a first area;
Fig. 10 is a perspective view of an ultrasound observation apparatus according to a first modification of the embodiment in which a shielding plate is provided on a right side surface; and
Fig. 11 is a perspective view of an ultrasound observation apparatus according to a second modification of the embodiment in which a shielding plate of an L shape in section is provided on a right side surface or a rear surface.

### DETAILED DESCRIPTION OF THE PREFERRED EMBODIMENTS

An embodiment of the present invention will be hereinafter explained with reference to the accompanying drawings. In the present embodiment, an ultrasound endoscope apparatus as an ultrasound medical apparatus and, in particular, an ultrasound observation apparatus are explained as examples.

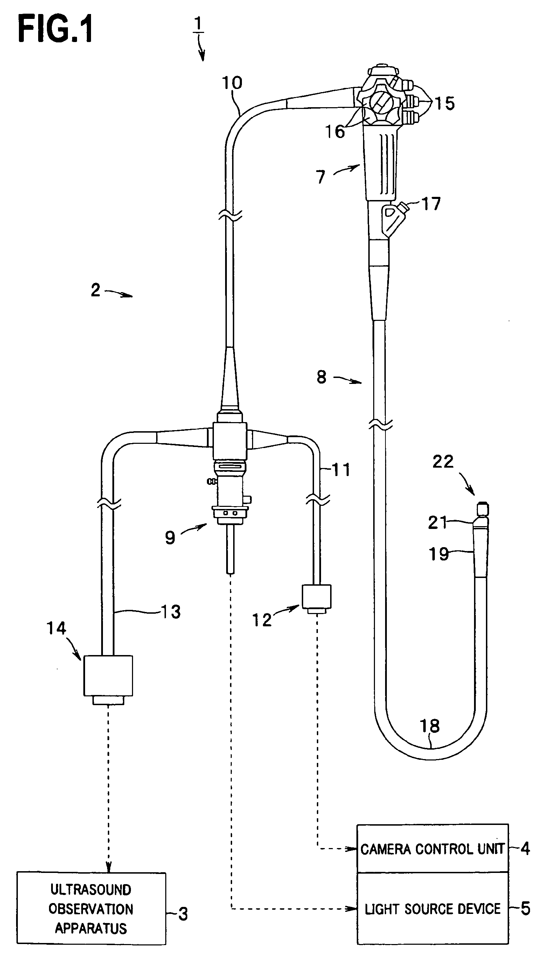

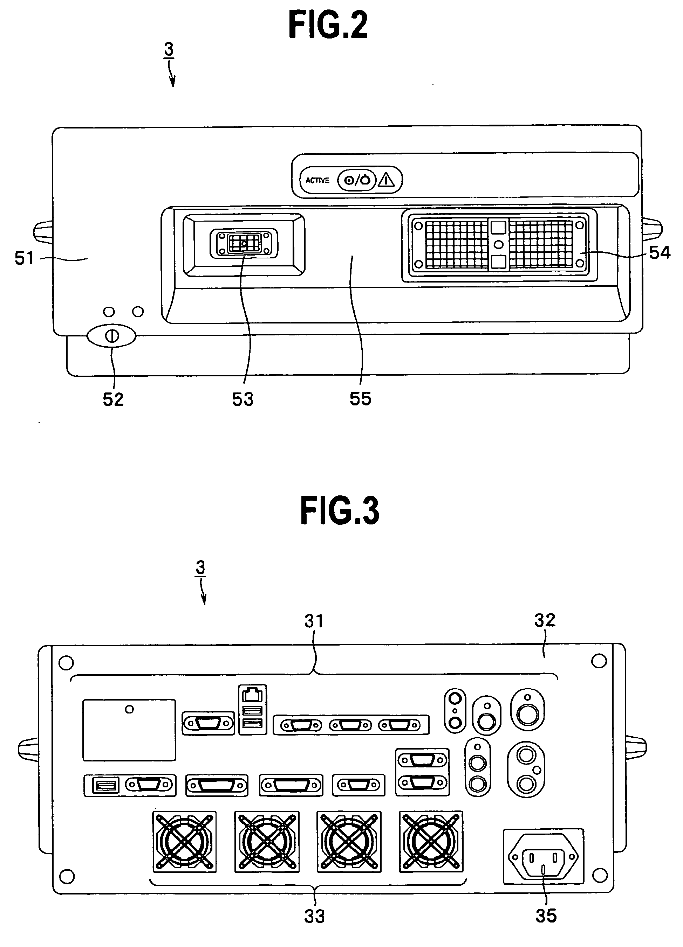

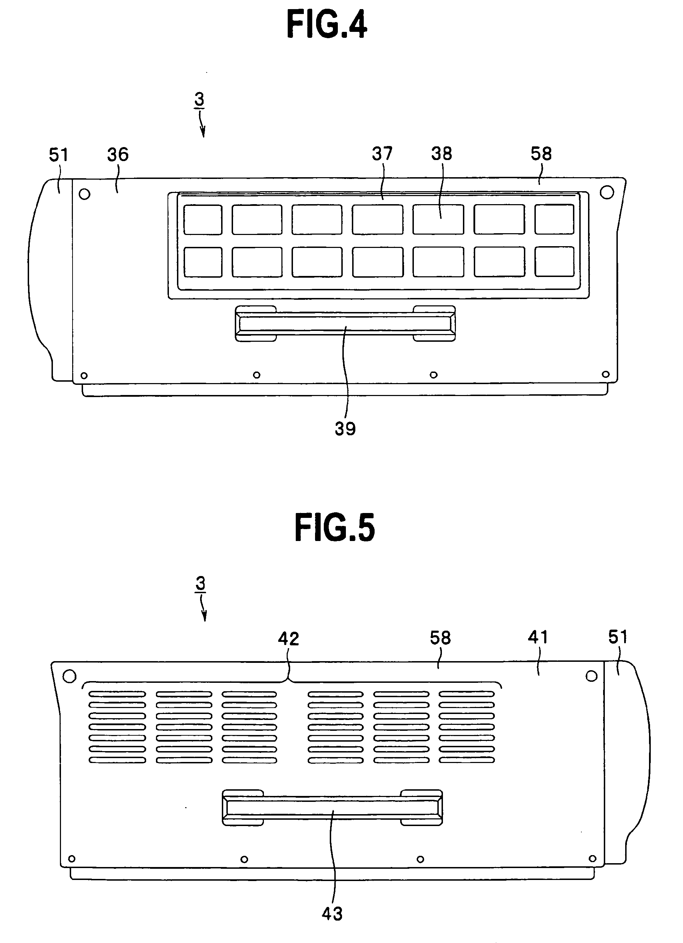

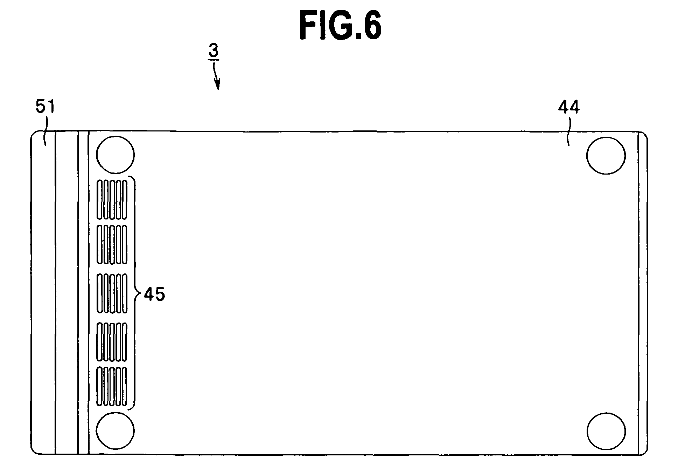

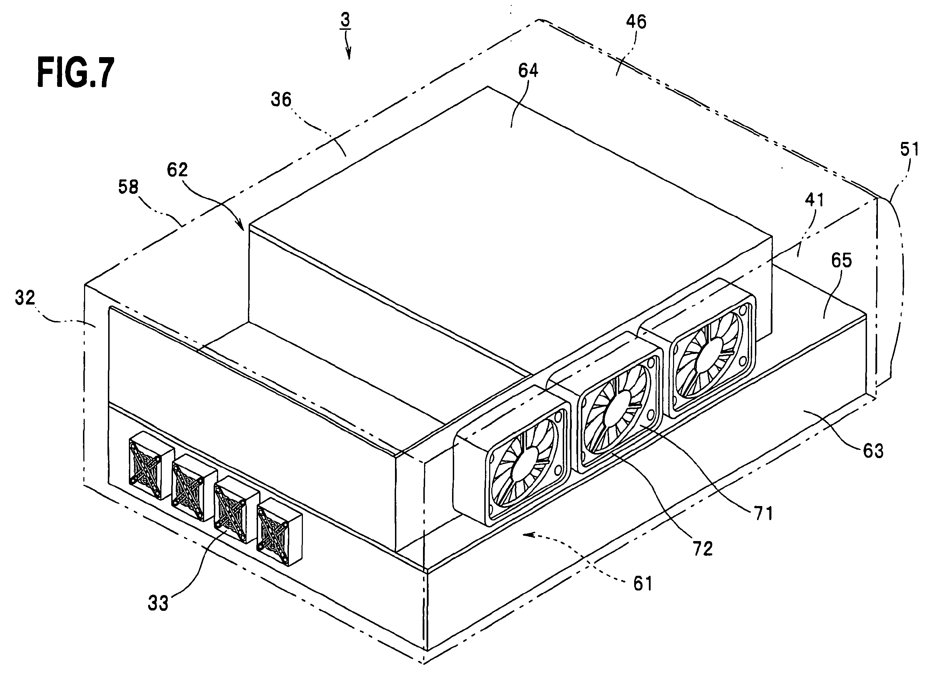

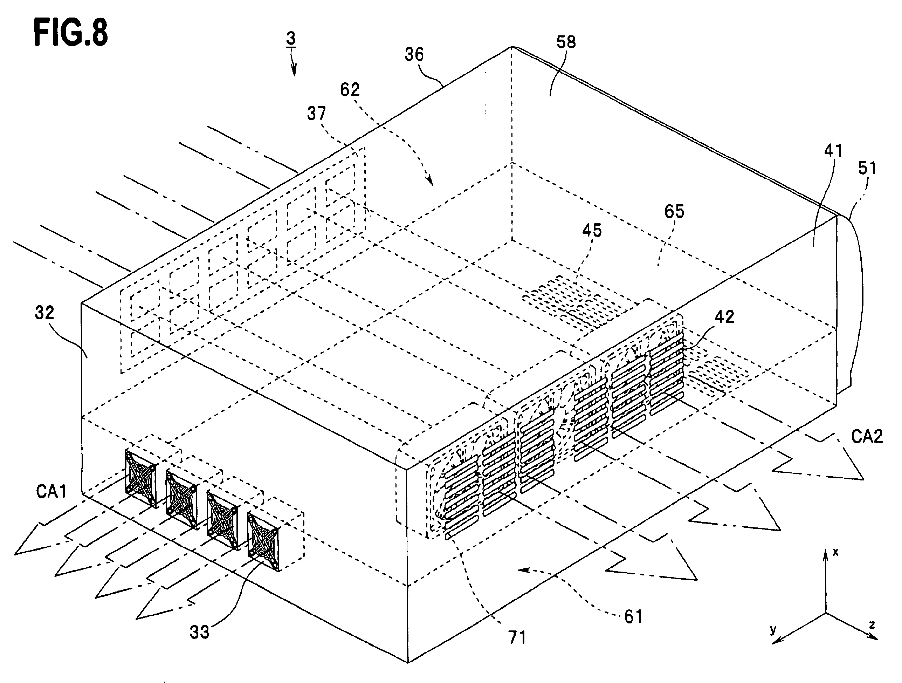

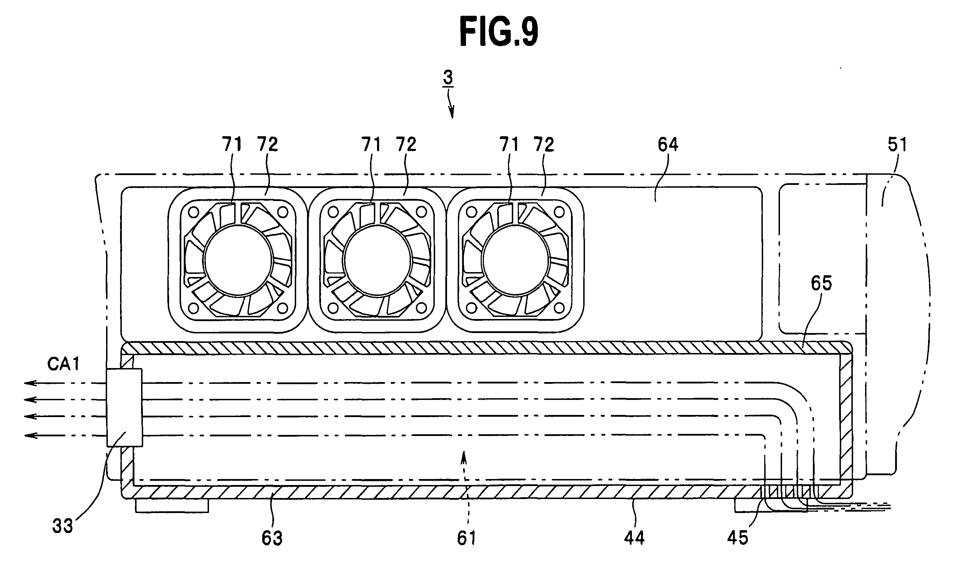

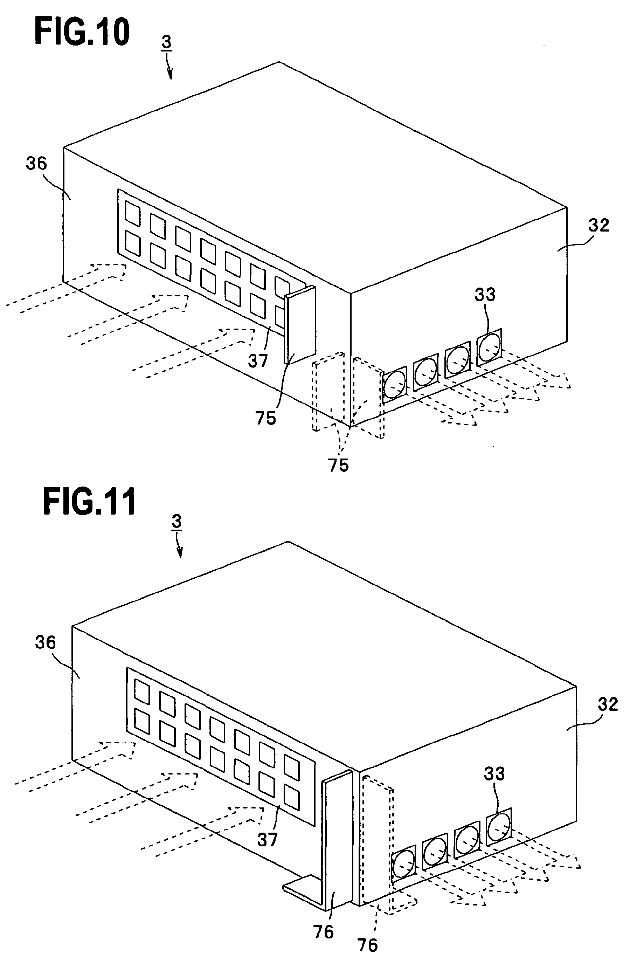

Figs. 1 to 11 show an embodiment of the present invention. Fig. 1 is a diagram for explaining the schematic configuration of an ultrasound endoscope. Fig. 2 is a front view of an ultrasound observation apparatus. Fig. 3 is a rear view of the ultrasound observation apparatus. Fig. 4 is a right side view of the ultrasound observation apparatus. Fig. 5 is a left side view of the ultrasound observation apparatus. Fig. 6 is a bottom view of the ultrasound observation apparatus. Fig. 7 is a perspective view showing the inside of the ultrasound observation apparatus. Fig. 8 is a perspective view for explaining air currents in the ultrasound observation apparatus caused by respective cooling fans. Fig. 9 is a partial sectional view for explaining air currents in the ultrasound observation apparatus caused by respective cooling fans that cool a first area. Fig. 10 is a perspective view of an ultrasound observation apparatus according to a first modification of the embodiment in which a shielding plate is provided on a right side surface. Fig. 11 is a perspective view of an ultrasound observation apparatus according to a second modification of the embodiment in which a shielding plate of an L shape in section is provided on a right side surface or a rear surface.

As shown in Fig. 1, an ultrasound endoscope apparatus 1 as an ultrasound medical apparatus according to the present embodiment mainly includes an ultrasound endoscope 2, an ultrasound observation apparatus 3, a camera control unit (hereinafter abbreviated as CCU) 4, and a light source device 5. The ultrasound observation apparatus 3 and the CCU 4 are connected to a not-shown monitor for displaying an ultrasound observation image and an endoscope image formed by the ultrasound endoscope 2.

The ultrasound endoscope 2 mainly includes an insertion section 8 inserted into a body cavity, an operation section 7 located at a proximal end portion of the insertion section 8, and a universal code 10 extended from a side of the operation section 7.

An endoscope connector 9 connected to the light source device 5 is provided at a proximal end portion of the universal code 10. From the endoscope connector 9, an electric cable 11 detachably connected to the CCU 4 via an electric connector 12 and an ultrasound cable 13 detachably connected to the ultrasound observation apparatus 3 via an ultrasound connector section 14 are extended.

The insertion section 8 of the ultrasound endoscope 2 is configured by consecutively providing, in order from a distal end side thereof, a distal end rigid portion 21 formed of a rigid resin member, a bendable bending portion 19 located at a rear end of the distal end rigid portion 21, and a flexible tube portion 18 that is slim and long and has flexibility, the flexible tube portion 18 being located at a rear end of the bending portion 19 and leading to a distal end portion of the operation section 7. An ultrasound transducer section 22 in which plural ultrasound transducers of an electronic scanning type for transmitting and receiving ultrasound are arrayed is provided on a distal end side of the distal end rigid portion 21.

An angle knob 16 for controlling bending of the bending portion 19 in a desired direction, various buttons 15 for performing air supply, water supply, suction operation, and the like, a treatment instrument insertion port 17 serving as an entrance of a treatment instrument introduced into a body cavity, and the like are provided in the operation section 7 of the ultrasound endoscope 2.

Although not shown in the figure, a lens cover for illumination that configures an illumination optical system, a lens cover for observation that configures an observation optical system, a forceps port also serving as a suction port, and an air-supply and water-supply nozzle are arranged on a distal end surface of the distal end rigid portion 21 on which the ultrasound transducer section 22 is provided. The distal end rigid portion 21 incorporates a not-shown image pickup means that is an image sensor such as a CCD or a CMOS for collecting photographing light led from the lens cover for observation and photoelectrically converting the photographing light.

The configuration of the ultrasound observation apparatus 3 to which the ultrasound endoscope 2 is connected is explained with reference to Figs. 2 to 7.

As shown in Fig. 2, the ultrasound observation apparatus 3 includes a power supply switch 52 disposed in a front panel 51 on a front surface thereof and first and second apparatus side ultrasound connector sections 53 and 54 of different types disposed in parallel in an apparatus side connector arrangement surface 55 formed in the front panel 51.

In the apparatus side connector arrangement surface 55, the first apparatus side ultrasound connector section 53 according to the present embodiment arranged on a left side on the figure is a plug connector of a 96-core type forming a pair with the second ultrasound connector section 14 of the ultrasound endoscope 2 described above.

On the other hand, in the apparatus side connector arrangement surface 55, the second apparatus side ultrasound connector section 54 according to the present embodiment arranged on a right side on the figure is a plug connector of a 260-core type forming a pair with an ultrasound connector of another ultrasound medical apparatus such as an ultrasound endoscope.

In a rear surface portion 32 of the ultrasound observation apparatus 3, plural connection terminals 31 to which plural communication cables and the like are connected, a power supply connector 35 to which a power supply cable is connected, and plural, in the present embodiment, four first cooling fans 33 are provided.

The plural connection terminals 31 are disposed on an upper side of the rear surface portion 32. The power supply connector 35 is disposed on the lower right of the rear surface portion 32. The four first cooling fans 33 are disposed in parallel on a lower side of the rear surface portion 32.

As shown in Fig. 4, in a right side surface portion 36 of the ultrasound observation apparatus 3, a tabular filter section 37 that configures an intake port and a handle section 39 grasped by a user when the user carries the ultrasound observation apparatus 3 are provided.

The filter section 37 is detachably disposed to hold dust collecting filters 38 and cover a not-shown opening for suction formed on an upper side of the right side surface portion 36. The filter section 37 has a magnet on a surface opposed to the right side surface portion 36, which is a metal armor member, and is held on the right side surface portion 36 by a magnetic force of the magnet. When the user performs replacement, cleaning, and the like of the dust collecting filters 38, the user can easily attach and detach the filter section 37 to and from the right side surface portion 36.

On the other hand, an exhaust port group 42 in which plural long holes are arranged in parallel and a handle section 43 that the user grasps in carrying the ultrasound observation apparatus 3 are provided in a left side surface portion 41 of the ultrasound observation apparatus 3.

The exhaust port group 42 is formed in an upper portion of the left side surface portion 41 and substantially opposed to an intake port (not shown) formed in the right side surface portion 36 to which the filter section 37 is attracted and fixed.

As shown in Fig. 6, in a lower surface portion 44 of the ultrasound observation apparatus 3, an intake port group 45 in which plural long holes are provided in parallel is provided near a front panel 51 on a front side.

A box-like external shape of the ultrasound observation apparatus 3 is formed by the front panel 51, the rear surface portion 32, both the side surface portions 36 and 41, the lower surface portion 44, and an upper surface portion 46 (see Fig. 7). An armor cover member 58 of a substantial C shape formed by both the side surface portions 36 and 41 and the upper surface portion 46 is detachably attachable to the ultrasound observation apparatus 3. The user can access contents of the ultrasound observation apparatus 3 in manufacturing, maintenance, and the like thereof by removing the cover member 58.

As shown in Fig. 7, the ultrasound observation apparatus 3 includes first and second heating areas 61 and 62 in which, although not shown in the figure, substrates on which power supply units or electronic components such as capacitors, AC/DC converters, regulators, and CPUs, which are changed to heating elements by electric power, are arranged are set.

Specifically, the first heating area 61 is an internal area of a first parts setting frame section 63 of a substantial box shape that configures a first heating element and is located on a lower side and opened on an upper side. In the first heating area, the not-shown power supply unit and electronic components are built.

More specifically, the first heating area 61 is an area formed by substantially closing an internal space of the first parts setting frame section 63 with a plate member 65 that closes an upper opening of the first parts setting frame section 63. In the first parts setting frame section 63, the four first cooling fans 33 are fixed to one surface opposed to the rear surface portion 32 of the ultrasound observation apparatus 3 to communicate with the first heating area 61 formed in the inside.

A bottom surface portion of the first parts setting frame section 63 configures the lower surface portion 44 of the ultrasound observation apparatus 3. The intake port group 45 configures an opening on a lower side of the first heating area 61. The first heating area 61 is preferably in a state in which a portion excluding the four first cooling fans 33 and the intake port group 45 is completely sealed.

However, in the first parts setting frame section 63, a hole portion is provided through which a communication cable for exchanging electric power supplied to the power supply unit therein and communication signals of the various electronic components with electronic components provided in the second heating area 62. Therefore, in the present embodiment, the first heating area 61 is not in the state in which the portion excluding the four first cooling fans 33 and the intake port group 45 is completely sealed. It is also possible that a connector is provided in the first parts setting frame section 63 or the plate member 65, a cable to which the electronic components provided in the second heating area 62 are electrically connected is connected to the connector, and the first heating area 61 is set in a state in which the portion excluding the four first cooling fans 33 and the intake port group 45 is completely sealed.

On the other hand, the second heating area 62 is, in the present embodiment, an area that is superimposed above the first heating area 61, configures a not-shown second heating element, and is surrounded by the plate member 65, the front panel 51, the armor cover member 58, and the rear surface portion 32 including an unsealed second parts setting frame section 64 of a substantial box shape in which electronic components of a transmission system are mainly arranged.

In the second parts setting frame section 64, three second cooling fans 71 that exhaust the air in a direction substantially orthogonal to an exhaust direction of the four first cooling fans 33 provided in the first parts setting frame section 63 are provided. The three second cooling fans 71 are arranged to be opposed to the exhaust port group 42 of the left side surface portion 41 of the armor cover member 58. In the three second cooling fans 71, sponges 72 with which a surface thereof opposed to an inner surface of the left side surface portion 41 of the armor cover member 58 comes into contact to surely discharge heat in the second heating area 62 from the exhaust port group 42 are provided in outer peripheral portions thereof, respectively.

Actions of the ultrasound observation apparatus 3 according to the present embodiment configured as explained above are explained with reference to Figs. 8 and 9.

As shown in Fig. 8, in the ultrasound observation apparatus 3 according to the present embodiment, the four first cooling fans 33 provided in the rear surface portion 32 are driven to suck and discharge heat in the first heating area 61 to the outside. Air currents in an arrow CA1 direction (a y axis direction in the figure) indicated by alternate long and two short dashes lines are generated in the first heating area 61. As for the exhaust currents for cooling the first heating area 61, as shown in Fig. 9, the external air is sucked into the first heating area 61 from the intake port group 45 formed in the lower surface portion 44 of the first parts setting frame section 63 near the front panel 51 by the driving of the four first cooling fans 33 and is exhausted through an exhaust path to the outside in the arrow CA1 direction from the rear surface portion 32.

Consequently, the first heating area 61 is cooled to optimum temperature without being heated by heat of the electronic components configuring the first heating element provided therein. As a result, a temperature environment in which the electronic components are driven normally is maintained.

Referring back to Fig. 8, in the ultrasound observation apparatus 3, the three second cooling fans 71 arranged to be opposed to the inner surface of the left side surface portion 41 are driven to suck and discharge heat in the second heating area 62 to the outside. Air currents in an arrow CA2 direction (a z axis direction in the figure) indicated by alternate long and two short dashes lines are generated in the second heating area 62. As for the air currents for cooling the second heating area 62, the external air is sucked from the filter section 37 of the right side surface portion 36 into the second heating area 62 by the driving of the three second cooling fans 71 and exhausted through an exhaust path to the outside in the arrow CA2 direction from the exhaust port group 42 of the left side surface portion 41.

Consequently, the second heating area 62 is cooled to optimum temperature without being heated by heat of the electronic components configuring the second heating element provided therein. As a result, a temperature environment in which the electronic components are driven normally is maintained.

For the respective air currents (the air currents in the arrow CA1 direction and the arrow CA2 direction), exhaust paths sectioned by the plate member 65 that partitions the first heating area 61 and the second heating area 62 are formed. Therefore, the air currents are prevented from being affected by reactions of the air currents each other. As a result, in the ultrasound observation apparatus 3 according to the present embodiment, the first and second heating areas 61 and 62 can be efficiently cooled. For the cooling fans 33 and 71, exhaust volumes necessary for cooling heat quantities of the electronic components of the heating areas 61 and 62 corresponding to the cooling fans 33 and 71, respectively, are set.

In the ultrasound observation apparatus 3 according to the present embodiment, the exhaust direction of the four first cooling fans 33 that discharge the heat in the first heating area 61 (the y axis direction as the arrow CA1 direction) and the exhaust direction of the three second cooling fans 71 that discharge heat in the second heating area 62 (the z axis direction as the arrow CA2 direction) are different directions, i.e., directions substantially orthogonal to each other. In other words, the heat discharged by the respective cooling fans 33 and 71 are not discharged in an identical direction.

Consequently, in the ultrasound observation apparatus 3 according to the present embodiment, air quantities of the respective cooling fans 33 and 71 are reduced and the exhaust directions of exhaust by the respective cooling fans 33 and 71 are different. Therefore, it is possible to prevent an unpleasant feeling from being given to a patient and a surgeon due to hot air directly or indirectly acting on the patient and the surgeon.

In the ultrasound observation apparatus 3 according to the present embodiment, as shown in Fig. 8, the four first cooling fans 33 are provided in the rear surface portion 32 and the second cooling fans 71 are provided on the inner surface side of the left side surface portion 41. In the ultrasound observation apparatus 3, the intake port group 45 communicating with the first heating area 61 is provided near the front panel 51 of the lower surface portion 44 and the filter section 37 of the intake port communicating with the second heating area 62 is provided in the right side surface portion 36.

According to an arrangement relation between the respective cooling fans 33 and 71 and the respective intake ports (the intake port group 45 and the filter section 37), in the ultrasound observation apparatus 3, the electronic components having a largest heating values are provided on the left side surface portion 41 side in the first heating area 61 and the electronic components having a largest heating value are provided on the rear surface portion 32 side in the second heating area 62. In the ultrasound observation apparatus 3, the cooling fans 33 and 71 that cool the electronic components having the largest heating values provided in the respective heating areas 61 and 62 are arranged to be most distant from the intake ports of the respective heating areas 61 and 62. In other words, in the ultrasound observation apparatus 3, an exhaust side having high temperature and a suction side are set in positions most distant from each other.

Consequently, in the respective heating areas 61 and 62, the exhausted hot air is prevented from refluxing to the intake ports. Therefore, cooling efficiency in the ultrasound observation apparatus 3 is improved because an arrangement of the exhaust side having highest temperature and the suction side is taken into account.

As shown in Fig. 10, a shielding plate 75 that shields exhaust from the first cooling fan 33 as a mere plate member indicated by a solid line may be provided on the rear surface portion 32 side of the right side surface portion 36 near the filter section 37, may be provided in the right side surface portion 36 near the first cooling fan 33 indicated by a broken line, or may be provided in the rear surface portion 32. The shielding plate 75 may be configured integrally with the filter section 37.

Moreover, as shown in Fig. 11, a shielding plate 76 of an L shape indicated by a solid line that shields exhaust from the first cooling fan 33 may be provided on the rear surface portion 32 side of the right side surface portion 36 near the filter section 37 or may be provided in the rear surface portion 32 near the right side surface portion 36 indicated by a broken line.

In this way, in the ultrasound observation apparatus 3, it is possible to easily prevent exhaust from refluxing to the suction side by providing the shielding plates 75 and 76.

In Figs. 10 and 11, the shielding plates 75 and 76 for preventing exhaust of the first cooling fan 33 from refluxing to the filter section 37 are shown. However, it goes without saying that the configuration of the shielding plates 75 and 76 can also be applied to the second cooling fan 71 side.

Moreover, in the present embodiment, the ultrasound endoscope apparatus 1 is explained as the example. However, it goes without saying that the embodiment can also be applied to other medical apparatuses.

The invention described in the embodiments is not limited to the embodiments and modifications thereof. Besides, it is possible to carry out various modifications at an implementation stage. Moreover, inventions at various stages are included in the embodiments. Various inventions can be extracted by appropriate combinations in the disclosed plural elements.

For example, even if several elements are deleted from all the elements described in the embodiments, when the problems described above can be solved and the effects described above can be obtained, a configuration obtained by deleting the elements can be extracted as an invention.

Having described the preferred embodiments of the invention referring to the accompanying drawings, it should be understood that the present invention is not limited to those precise embodiments and various changes and modifications thereof could be made by one skilled in the art without departing from the scope of the invention as defined in the appended claims.

## Claims

1. An ultrasound medical apparatus (3) to which an ultrasound endoscope is to be connected comprising: a housing having a front panel (51), a rear surface portion (32), side surface portions (36, 41), lower surface portion (44) and cover member (58);
a first heating area (61) in the housing in which a first heating unit is provided;
a second heating area (62) in the housing that is arranged to be superimposed on the first heating area (61) by being partitioned into a space different from the first heating area (61) and in which a second heating unit is provided;
a first cooling fan (33) that is provided at a rear surface portion (32) of the housing and configured to discharge heat in the first heating area (61) through the rear surface portion (32) to the outside of the first heating area (61) by generating an air current in a first direction (CA1) within the first heating area (61); and **characterised by**
a second cooling fan (71) that is arranged to be opposed to an exhaust port group (42) on one side surface portion (41), which is orthogonal to the rear surface portion (32) of the housing, and configured to discharge heat in the second heating area (62) through the exhaust port group (42) to the outside of the second heating area (62) by generating an air current in a second direction (CA2) within the second heating area (62) that is substantially orthogonal to the first direction (CA1) of the air current generated by the first cooling fan (33),
wherein a direction in which the heat from the first heating area (61) is discharged through the rear surface portion (32) and a direction in which the heat in the second heating area (62) is discharged through the exhaust port group (42) are substantially orthogonal to each other..

2. The ultrasound medical apparatus according to claim 1, further comprising plate member (65) that partitions the first heating area (61) and the second heating area (62) to shield the air currents generated by the first cooling fan (33) and the second cooling fan (71) from each other.

3. The ultrasound medical apparatus according to claim 1 or 2, wherein a shielding plate (75, 76) is provided between an exhaust section of the first cooling fan (33) and a suction side of the second cooling fan (71) to prevent the second cooling fan (71) from sucking exhaust by the first cooling fan (33).

4. The ultrasound medical apparatus according to claim 3, wherein the shielding plate (75, 76) is provided on an exhaust section side of the first cooling fan (33) or the second cooling fan (71).

5. The ultrasound medical apparatus according to any one of claims 1 to 4, wherein
the medical apparatus (3) has a plurality of the first cooling fans (33); and
the plurality of the first cooling fans (33) are disposed in parallel in a direction orthogonal to a direction of superimposition of the first heating area (61) and the second heating area (62).

6. The ultrasound medical apparatus according to any one of claims 1 to 5, wherein
the medical apparatus (3) has a plurality of the second cooling fans (71); and
the plurality of the second cooling fans (71) are disposed in parallel in a direction orthogonal to a direction of superimposition of the first heating area (61) and the second heating area (62).

## Patentansprüche

1. Medizinisches Ultraschallgerät (3), mit dem ein Ultraschallendoskop verbindbar ist, umfassend:
ein Gehäuse, das eine Frontblende (51), einen Rückseitenabschnitt (32), Seitenflächenabschnitte (36, 41), einen Bodenflächenabschnitt (44) und ein Abdeckelement (58) umfasst;
einen in dem Gehäuse angeordneten ersten Heizbereich (61), in dem eine erste Heizvorrichtung vorhanden ist;
einen in dem Gehäuse angeordneten zweiten Heizbereich (62), der so angeordnet ist, dass er über dem ersten Heizbereich (61) liegt, indem er in einem anderen Bereich abgetrennt ist als der erste Heizbereich (61) und in dem eine zweite Heizvorrichtung vorhanden ist;
ein erstes Kühlgebläse (33), das an dem Rückseitenabschnitt (32) des Gehäuses vorgesehen und dazu eingerichtet ist, Wärme in dem ersten Heizbereich (61) durch den Rückseitenabschnitt (32) an die Außenumgebung des ersten Heizbereichs (61) abzuführen, indem es einen Luftstrom in eine erste Richtung (CA1) innerhalb des ersten Heizbereichs (61) erzeugt; und
**gekennzeichnet durch**
ein zweites Kühlgebläse (71), das so angeordnet ist, dass es einer Gruppe von Ausströmöffnungen (42) an einem Seitenflächenabschnitt (41) gegenüberliegt, der orthogonal zu dem Rückseitenabschnitt (32) des Gehäuses angeordnet ist, und das dazu eingerichtet ist, Wärme in dem zweiten Heizbereich (62) **durch** die Gruppe von Ausströmöffnungen (42) an die Außenumgebung des zweiten Heizbereichs (62) abzuführen, indem es einen Luftstrom in eine zweite Richtung (CA2) innerhalb des zweiten Heizbereichs (62) erzeugt, die im Wesentlichen orthogonal zu der ersten Richtung (CA1) des Luftstroms ist, der **durch** das erste Kühlgebläse (33) erzeugt wird,
wobei eine Richtung, in welche die Wärme von dem ersten Heizbereich (61) durch den Rückseitenabschnitt (32) abgeführt wird
und eine Richtung, in welche die Wärme in dem zweiten Heizbereich (62) durch die Gruppe von Ausströmöffnungen (42) abgeführt wird
im Wesentlichen orthogonal zueinander sind.

2. Medizinisches Ultraschallgerät gemäß Anspruch 1, weiterhin umfassend ein Plattenelement (65), das den erste Heizbereich (61) und den zweiten Heizbereich (62) voneinander trennt, um die durch das erste Kühlgebläse (33) und das zweite Kühlgebläse (71) erzeugten Luftströme voneinander abzuschirmen.

3. Medizinisches Ultraschallgerät gemäß Anspruch 1 oder 2, wobei eine Abschirmplatte (75, 76) zwischen einem Auslassabschnitt des ersten Kühlgebläses (33) und einer Saugseite des zweiten Kühlgebläses (71) vorgesehen ist, um zu verhindern, dass das zweite Kühlgebläse (71) Abgase des ersten Kühlgebläses (33) ansaugt.

4. Medizinisches Ultraschallgerät gemäß Anspruch 3, wobei die Abschirmplatte (75, 76) auf einer Auslassabschnittseite des ersten Kühlgebläses (33) oder des zweiten Kühlgebläses (71) vorgesehen ist.

5. Medizinisches Ultraschallgerät gemäß einem der Ansprüche 1 bis 4, wobei
das medizinische Gerät (3) eine Mehrzahl von ersten Kühlgebläsen (33) aufweist; und
die Mehrzahl von ersten Kühlgebläsen (33) in einer Richtung orthogonal zu einer Überlagerungsrichtung des ersten Heizbereichs (61) und des zweiten Heizbereichs (62) parallel zueinander angeordnet sind.

6. Medizinisches Ultraschallgerät gemäß einem der Ansprüche 1 bis 5, wobei
das medizinische Gerät (3) eine Mehrzahl von zweiten Kühlgebläsen (71) aufweist; und
die Mehrzahl von zweiten Kühlgebläsen (71) in einer Richtung orthogonal zu einer Überlagerungsrichtung des ersten Heizbereichs (61) und des zweiten Heizbereichs (62) parallel zueinander angeordnet sind.

## Revendications

1. Appareil médical à ultrasons (3) auquel un endoscope ultrasonore est destiné à être connecté comprenant :
un logement comprenant un panneau avant (51), une partie de surface arrière (32), des parties de surfaces latérales (36, 41), une partie de surface inférieure (44) et un élément de couvercle (58) ;
une première zone de chauffage (61) dans le logement dans laquelle une première unité de chauffage est prévue ;
une deuxième zone de chauffage (62) dans le logement qui est agencée pour être superposée sur la première zone de chauffage (61) en étant partitionnée en un espace différent de la première zone de chauffage (61) et dans laquelle une deuxième unité de chauffage est prévue ;
un premier ventilateur de refroidissement (33) qui est prévu au niveau d'une partie de surface arrière (32) du logement et configuré pour évacuer la chaleur dans la première zone de chauffage (61) par l'intermédiaire de la partie de surface arrière (32) vers l'extérieur de la première zone de chauffage (61) en générant un courant d'air dans une première direction (CA1) à l'intérieur de la première zone de chauffage (61) ; et **caractérisé par**
un deuxième ventilateur de refroidissement (71) qui est agencé pour être opposé à un groupe d'orifices d'échappement (42) sur une partie de surface latérale (41), qui est orthogonale à la partie de surface arrière (32) du logement, et configuré pour évacuer la chaleur dans la deuxième zone de chauffage (62) par l'intermédiaire du groupe d'orifices d'échappement (42) vers l'extérieur de la deuxième zone de chauffage (62) en générant un courant d'air dans une deuxième direction (CA2) à l'intérieur de la deuxième zone de chauffage (62) qui est sensiblement orthogonale à la première direction (CA1) du courant d'air généré par le premier ventilateur de refroidissement (33),
dans lequel une direction dans laquelle la chaleur provenant de la première zone de chauffage (61) est évacuée par l'intermédiaire de la partie de surface arrière (32), et une direction dans laquelle la chaleur dans la deuxième zone de chauffage (62) est évacuée par l'intermédiaire du groupe d'orifices d'échappement (42), sont sensiblement orthogonales l'une par rapport à l'autre.

2. Appareil médical à ultrasons selon la revendication 1, comprenant en outre un élément de plaque (65) qui partitionne la première zone de chauffage (61) et la deuxième zone de chauffage (62) pour protéger les courants d'air générés par le premier ventilateur de refroidissement (33) et le deuxième ventilateur de refroidissement (71) l'un de l'autre.

3. Appareil médical à ultrasons selon la revendication 1 ou 2, dans lequel une plaque de blindage (75, 76) est prévue entre une section d'échappement du premier ventilateur de refroidissement (33) et un côté aspiration du deuxième ventilateur de refroidissement (71) pour empêcher le deuxième ventilateur de refroidissement (71) d'aspirer les échappements du premier ventilateur de refroidissement (33).

4. Appareil médical à ultrasons selon la revendication 3, dans lequel la plaque de blindage (75, 76) est prévue sur un côté section d'échappement du premier ventilateur de refroidissement (33) ou du deuxième ventilateur de refroidissement (71).

5. Appareil médical à ultrasons selon l'une quelconque de revendication 1 à 4, dans lequel
l'appareil médical (3) comporte une pluralité de premiers ventilateurs de refroidissement (33) ; et
la pluralité de premiers ventilateurs de refroidissement (33) sont agencés en parallèle dans une direction orthogonale à une direction de superposition de la première zone de chauffage (61) et de la deuxième zone de chauffage (62).

6. Appareil médical à ultrasons selon l'une quelconque des revendications 1 à 5, dans lequel
l'appareil médical (3) comporte une pluralité de deuxièmes ventilateurs de refroidissement (71) ; et
la pluralité de deuxièmes ventilateurs de refroidissement (71) sont agencés en parallèle dans une direction orthogonale une direction de superposition de la première zone de chauffage (61) et de la deuxième zone de chauffage (62).
